# EUROPEAN PATENT APPLICATION

(11) **EP 2 218 455 A1**
(43) Date of publication of application: **18.08.2010**
(21) Application number: 09001741.9
(22) Date of filing: 07.02.2009
(51) Int. Cl.: A61K 36/48, A61P 17/00

(54) **Dolichos biflorus extract for use in therapeutic skin treatment**

(71) Applicant: Cognis IP Management GmbH, 40589 Düsseldorf (DE)
(72) Inventor: Moussou, Philippe, 54510 Tomblaine (FR); Moser, Philippe, 54270 Essey Les Nancy (FR); Jeanmaire, Christine, 54000 Nancy (FR); Danoux, Louis, 54420 Saulxures les Nancy (FR); Bardey, Vincent, 54000 Nancy (FR)

(57) **Abstract**

The present invention pertains to a *Dolichos biflorus* extract enhancing collagen production and a pharmaceutical composition comprising said extract. Furthermore, it pertains to the use of said extract or composition as a medicament, especially to their use in enhancing collagen production. Finally, a method for preparing said extract and for its therapeutic use is described. The *Dolichos biflorus* extract is preferably made from seeds by alcoholic or hydroalcoholic extraction.

## Description

### Technical Field

The present invention pertains to a *Dolichos biflorus* extract enhancing collagen production and a pharmaceutical composition comprising said extract. Furthermore, it pertains to the use of said extract or composition as a medicament, especially to their use in enhancing collagen production. Finally, a method for preparing said extract and for its therapeutic use is described. The *Dolichos biflorus* extract is preferably made from seeds by alcoholic or hydroalcoholic extraction.

### Prior Art

There are a number of pharmaceutical compositions available to consumers to improve the health of the skin and to support wound healing.

The skin consists of three layers, the epidermis, the dermis and the subcutaneous tissue (hypodermis). The skin's extracellular matrix is a complex network of macromolecules, such as collagen or elastic fibers, glycoproteins, glycosaminoglycans and proteoglycans. It provides a physical framework to assume mechanical strength and participates in cell metabolism regulation.

The dermis is composed of two distinct layers, the papillary and reticular dermis.

In the dermis more than 70% of the proteins are collagens. Collagen is responsible for the skin's strength. Collagen is produced by cells called fibroblasts, which are found scattered throughout the dermis.

One major type of collagen in the human skin dermis is type I collagen, which forms a network of fibers.

The dermis also contains quantitatively minor collagens, including type V collagen. This collagen plays an important role in the type I collagen fiber initiation and size control. Type V collagen interacts with the core of on-growing collagen I fibrils to favor the fiber formation. In addition, type V collagen induces a steric constraint that limits the growth of collagen fibers and so regulates their diameter.

Several ingredients claim the stimulation of collagen I synthesis, but without any effect on type V collagen. This could induce the formation of low quality collagen fiber networks.

Other minor collagens are present in the dermis, such as e.g. type III, VI, VII, XII, XIV, XVI, XVIII and XIX collagens. All these collagens participate to build a functional dermis.

The type IV collagen is present at the level of basement membrane. In the skin, it is particularly expressed at the level of the dermo-epidermal junction. The increase of the collagen IV can efficiently improve the quality of the dermo-epidermal junction.

The enhancement of collagens production is also desirable in certain collagen disorders, e.g. in classical Ehlers-Danlos syndrome (EDS). In said variety of EDS, the production of collagen I and V is impaired. One result of this impairment is a tendency of the skin to tear, bruise and to heal slowly.

*Dolichos biflorus* is a member of the cowpea family, also named Southern pea or Italian cowpea. Its botanical name is *Vigna unguiculata subsp. unguiculata* cv-gr Biflora Westphal.

It is an herbaceous, prostrate, climbing, or sub-erect to erect annual, growing 0.3 to 4 m long. Leaves are alternate, trifoliolate with petioles 5-25 cm long. The lateral leaflets are opposite and asymmetrical, while the top leaflet is symmetrical. Pods are pendent or erect to spreading, linear. Seeds are variable in size and shape, square to oblong and variously coloured (white, brown, maroon, cream and green). *Dolichos biflorus* is grown in India and Sri Lanka for seeds and as a vegetable. Tender green pods are consumed as green vegetable. Dried seeds are used whole or split. It makes excellent forage. Furthermore, *D. biflorus* is used in traditional ayurvedic medicine, e.g. as antilithic.

The cowpea (*Vigna unguiculata*) is one species from the genus Vigna, belonging to the Fabaceae family. In the literature, some uses for Vigna species are described, but not for *D*. *biflorus:*

WO 2006/053415 (Biopharmacopae) discloses plant extracts, *inter alia* Vigna extracts, having a matrix metalloprotease (MMP) inhibiting activity, and their dermatological use. The MMP inhibition shown in WO 2006/053415 may lead to reduced degradation of components of the extracellular matrix of the skin. The Vigna species listed in WO 2006/053415 show only low to moderate MMP inhibition; thus, Vigna does not belong to the preferred extracts used for skin treatment in WO 2006/053415. The preferred extracts described in WO 2006/053415 are used for the treatment or prevention of several skin conditions like wrinkling and sagging of the skin and irradiation induced skin damages, or for routine skin care.

WO 2005/058476 (GAT Formulation GmbH) describes a microencapsulation process for encapsulation of cosmetic ingredients. One source of these ingredients may be a *Dolichos biflorus* extract.

WO 02/055049 (Cognis France & YSL Beautè) describes cosmetic and pharmaceutical preparations for skin protection against dehydration. Said preparations comprise Late Embryogenesis Abundant (LEA) proteins. Cowpea extracts are mentioned as one source of LEA proteins.

None of these WO documents mentions *D*. *biflorus* seeds as a source of an extract suitable for a pharmaceutical composition or medicament for skin treatment, let alone for collagen enhancement.

Aim of present invention was to provide a safe product for therapeutic skin treatment in order to improve the mechanical strength and integrity of the skin. A particular aim was to provide a product that enhances collagen production, preferably collagen I, collagen III, collagen IV, collagen V, collagen VI, collagen VII, collagen XII, collagen XIV, collagen XVI, collagen XVIII, and/or collagen XIX production, more preferably collagen I and/or V production, in order to achieve *de novo* synthesis of a high quality collagen network in skin. In particular, therapeutic effects of collagen production enhancement on injured skin, e.g. bruised or cut skin, and on collagen disorders, especially on skin collagen disorders, are desired.

A further particular aim of present invention was to provide a product which contributes to the skin's health by other means, especially by improving the metabolism of the skin cells.

These and other objectives as they will become apparent from the ensuing descripttion of the invention are solved by the present invention as described in the independent claims. The dependent claims relate to preferred embodiments.

### Summary of the Invention

The present invention pertains to a *Dolichos biflorus* extract and its use, especially in as a medicament in therapeutic skin treatment.

The *D. biflorus* extract is particularly used for preparing a medicament for therapeutic skin treatment, especially for the treatment of collagen disorders and for wound healing.

Surprisingly, it was found that a *D. biflorus* extract can enhance the level of collagen in skin and skin cells, especially the level of collagen produced by human dermal fibroblasts, particularly the level of collagen I and/or V. This may have a beneficial effect on the formation and composition of the skin collagen matrix network.

Thus, the present invention is based on the ability of a *Dolichos biflorus* extract to improve collagen production, preferably the production of collagen I, III, IV, V, VI, VII, XII, XIV, XVI, XVIII and/or XIX, more preferably the production of collagen I and/or V, and even more preferably of both collagen I and V, for achieving a well organized skin collagen matrix network. In a use of the extract as a medicament, this ability may be exploited for treatment of a collagen disorder and/or improvement of wound healing.

It was furthermore found that a *D. biflorus* extract can improve the cell metabolism of a skin cell (particularly a fibroblast), especially its energetic metabolism (particularly by increasing its ATP synthesis rate). This ability may also be exploited in therapeutic skin regeneration, especially in wound healing.

Moreover, it was found that the *D. biflorus* extract according to present invention has additional skin protecting and skin regenerating properties which are advantageous in a medicament for skin treatment. It acts as antioxidant and/or UV-protective factor.

In particular, the present invention provides the following embodiments:
(1) a *Dolichos biflorus* extract for use as a medicament for enhancing collagen production, particularly collagen I and/or V production in the skin;
(2) the *Dolichos biflorus* extract according to (1) above, wherein the medicament is for treating a collagen disorder or for wound healing;
(3) the *Dolichos biflorus* extract according to (1) or (2) above, which
   (i) is an extract of a seed or a seed containing plant part, and/or
   (ii) is an alcoholic or hydroalcoholic extract;
(4) the use of the *Dolichos biflorus* extract for the preparation of a medicament for enhancing collagen production in the skin;
(5) a pharmaceutical composition comprising the *Dolichus biflorus* extract; and
(6) a method for preparing the *D. biflorus* extract, comprising a step wherein a *D*. *biflorus* whole plant or plant part is extracted (extraction step), preferably an alcoholic or hydroalcoholic extraction step.

### Detailed Description of the Invention

The present invention relates to a *Dolichos biflorus* extract, particularly to a *Dolichos biflorus* extract which has the ability to enhance collagen production and/or to enhance cell metabolism in the skin or skin cells. The invention furthermore relates to pharmaceutical compositions comprising said extract. Said extract is preferably made from raw *Dolichos biflorus* plant material, particulary plant material comprising *D*. *biflorus* seeds. Furthermore, the present invention pertains to the use of said *Dolichos biflorus* extract or compositions for therapeutic purposes, i.e. as a medicament, especially their use in enhancing collagen production in skin and/or skin cells.

The *Dolichos biflorus* extract is prepared by a method comprising at least one extraction step. Preferably, the *Dolichos biflorus* raw plant material is extracted by aqueous, alcoholic or hydroalcoholic extraction, resulting in an extract which is able to enhance collagen production and/or acts as metabolism enhancer.

### Terms and definitions

As used in the context of present invention, the singular forms of "a" and "an" also include the respective plurals unless the context clearly dictates otherwise. Thus, the term "a" or "an" is intended to mean "one or more" or "at least one", unless indicated otherwise.

The term "about" in context with a numerical value or parameter range denotes an interval of accuracy that the person skilled in the art will understand to still ensure the technical effect of the feature in question. The term typically indicates deviation from the indicated numerical value of +/- 10 %, preferably +/- 5 %.

"Comprising" in the context of present invention means that the components listed after the word "comprising" are either the sole components forming the composition (i.e. the composition consist of said components), or that in addition to said components at least one further component is present. In a preferred meaning, it means "consisting essentially of", and in its narrowest meaning, it is synonymous to "consisting of."

The term "pharmaceutical composition" designates either a medicament or a composition suitable for preparation of a medicament. Typically, the term designates a medicament.

The term "topical" as used herein includes any route of administration that enables the administrated compound or composition to line the tissue to which it is administrated (e.g. a skin surface). It includes any route of administration other than parenteral or per os. It includes, for example, spray application, smearing or soaking.

The term "extract" is intended to pertain both to the liquid resulting from the extraction step according to present invention and to the intermediate and final product (which typically is a dry product) of the process for preparation of the *D. biflorus* extract according to present invention. The appropriate meaning is generally clear from the context. Each of the liquid extract, the intermediate and the final product may be used in the context of present invention, e.g. in a composition or use according to present invention. But the final product, especially the dried final product or a composition comprising said final product is preferred.

The term "plant part" refers to any part or parts of a plant taken either individually or in a group. Examples include leaves, flowers, roots, seeds, pods, stems, fruits, seed coats, buds and other parts of a plant. In the context of present invention, seeds and seed containing plant parts are preferred.

The term "skin" refers to the skin of an animal or human, preferably a mammalian skin or - even more preferred - human skin. "Skin" encompasses the epidermis, the dermis and the hypodermis. The term "skin cells" pertains to any cell typically present in the skin, e.g. a cell of the group consisting of keratinocytes, fibroblasts, endothelial cells, basal cells, granular cells, Merkel cells, melanocytes, Langerhans cells, leukocytes, mastocytes, nerve cells, adipose cells and macrophages. In the context of present invention, fibroblasts and/or keratinocytes are preferred, and fibroblasts are particularly preferred. Furthermore, human skin cells, particularly human fibroblasts and/or keratinocytes are preferred.

The term "ameliorate" or "amelioration" includes the arrest, prevention, decrease, or improvement in one or more of the signs, features or symptoms of the indicated condition, may it be temporary or long-term.

"Enhancing collagen production" or "enhancement of collagen production" in the context of present invention pertains to any enhancement of collagen production by application or administration of a *D*. *biflorus* extract to a biological sample or human or animal subject in comparison to an untreated control, particularly to increasing the synthesis rate of collagen or the amount or concentration of collagen in a cell or tissue, especially in a skin cell or skin. When compared to an untreated control, the amount of collagen production in the treated tissue or cell is typically more than 100%, and preferably is at least 110%, at least 130%, at least 150% or at least 170% of the collagen production in the untreated control. In a preferred aspect, "enhancing collagen production" encompasses or - more preferably - means "enhancing collagen synthesis". Enhancement of collagen production may be achieved by increasing the collagen amount or concentration in a cell, by increasing the collagen release and/or production rate of a cell, by increasing the collagen amount synthesized per time (collagen synthesis rate), by increasing the concentration of collagen synthesizing enzymes, and/or by increasing the translation and/or transcription products of genes encoding collagen synthesizing enzymes. The collagen production enhancement may take place in any tissue or cell type, but skin and skin cells are preferred. In a specific embodiment, the collagen production is enhanced in a fibroblast or keratinocyte, preferably in a skin fibroblast or epidermal keratinocyte. The enhancement of collagen production, particularly of collagen synthesis, is verifiable by any method for collagen quantification or quantification of collagen synthesizing enzymes, especially by one of the methods described in the Examples.

The term "hydroalcoholic" is synonymous to "aqueous-alcoholic".

A "low molecular weight alcohol" is an alcohol having 1 to 5 carbon atoms and may be a primary, secondary or tertiary alcohol. It may be a mono- or polyalcohol, with mono-, di- and trialcohols being preferred, monoalcohols, glycols and glycerine being especially preferred. Accordingly, the low molecular weight alcohol may preferably be selected from the group consisting of methanol, ethanol, 1-propanol, 1-butanol, 2-propanol, 2-butanol, 2-methyl-1-propanol, 2-methyl-2-propanol, pentanol, glycerine, ethylene glycol, diethylene glycol, dipropylene glycol, propylene glycol, 1,3-butylene glycol, pentylene glycol, and mixtures thereof. In the context of present invention methanol, ethanol, propylene glycol, butylene glycol, and pentylene glycol are especially preferred.

### Dolichos biflorus Extract

### The present invention pertains to a D. biflorus extract and its use.

Generally any and all parts of the *D. biflorus* plant can be used as raw material to prepare an extract according to the invention. Entire plants or plant parts (like seeds), fresh or dried, may be used as starting material, which may be mechanically size-reduced before extraction. In a preferred aspect a seed or seed containing plant part, more preferably a seed, is used as starting material. Particularly preferred are dried seeds. In one preferred aspect, the seeds are mechanically size-reduced before extraction, e.g. by splitting, grinding or milling. The product of this size reduction is also a preferred raw material for preparing the *D. biflorus* extract according to present invention.

The *D. biflorus* plant or plant parts may be fresh or pretreated before their extraction. Pretreatment methods are preferably selected from the group consisting of drying, freeze-drying, freezing, mechanical size reduction, and combinations thereof. The plant material used as raw material for extraction can be fresh, dried or frozen. Besides mechanical size reduction, drying and freeze-drying are the most preferred pretreatment methods.

The plant material may be used immediately after harvesting or it can be stored for a period of time prior to being subjected to the extraction process, with storage being preferred to immediate use after harvesting. Fresh plants or plant parts may be used as the starting material although dried plants and/or plant parts - which may be mechanically size-reduced before extraction - are normally used.

Consequently, the *D. biflorus* extract is preferably prepared from dried plant parts, most preferably from dried seeds or dried plant parts containing seeds. Even more preferentially grinded dried seeds are used.

The *D. biflorus* extract does preferably have a reduced LEA content (e.g. reduced in comparison to the total LEA content in the raw material), or it is LEA free. The LEA content of the extract may be determined as described in WO 02/055049.

The *D. biflorus* extract typically is an aqueous, alcoholic or hydroalcoholic extract, preferably an alcoholic or hydroalcoholic extract.

A *D. biflorus* extract which (i) is an extract of a seed or a seed containing plant part, and/or (ii) is an alcoholic or hydroalcoholic extract is an especially preferred extract in the context of present invention, with a *D. biflorus* extract which (i) is an extract of a seed or a seed containing plant part, and (ii) is an alcoholic or hydroalcoholic extract being particularly preferred. In a specific embodiment, the *D. biflorus* extract in the context of present invention is a hydroalcoholic (e.g. a water/ethanol) seed extract.

The *D. biflorus* extract according to present invention is in one embodiment an extract which enhances collagen production, i.e. it acts as a collagen production enhancer. Preferably, it enhances the production of one or more collagens selected from the group consisting of collagen I, III, IV, V, VI, VII, XII, XIV, XVI, XVIII and XIX, more preferably the production of collagen I and/or V, and particularly the production of collagen I and V.

The *D. biflorus* extract according to present invention is in an even further embodiment a metabolism enhancer, i.e. it acts as a metabolism enhancer. It is able to increase the metabolic rate, particularly the ATP synthesis rate in skin cells, especially in fibroblasts (compare Examples).

Preferably, the *D. biflorus* extract according to present invention possesses both collagen production enhancing activity and a metabolism enhancing activity.

The *D. biflorus* extract according to present invention may also act as a UV-protective factor. It is able to confer UV protection to skin and skin cells, especially to keratinocytes and fibroblasts (compare Examples).

The *D. biflorus* extract according to present invention may also act as an antioxidant. It is able to confer protection against oxidative stress to skin and skin cells, especially to keratinocytes and fibroblasts (compare Examples).

The *D. biflorus* extract according to present invention may also act as an antiinflammatory agent. It is able to reduce the inflammatory response in cells, especially in skin cells like keratinocytes and fibroblasts (compare Examples).

Preferably, the *D. biflorus* extract according to present invention possesses more than one of the activities listed above.

The *D. biflorus* extract according to the invention or a pharmaceutical composition comprising said extract is preferably used as a medicament as described below in the section "Use of the *D. biflorus* extract and composition", particularly as a medicament for therapeutic skin treatment. One preferred use is for enhancement of collagen production, preferably of the production of one or more collagens selected from the group consisting of collagen I, III, IV, V, VI, VII, XII, XIV, XVI, XVIII and XIX, more preferably of collagen I and/or V. Particularly preferred is the therapeutic use for enhancement of collagen I and V production. Another preferred therapeutic use is the use of the *D. biflorus* extract as a metabolism enhancer.

The effects of the *D. biflorus* extract on skin and skin cells encompass the improvement of the skin firmness, increasing the skin's regenerative and renewal process, and/or to strengthen the skin extra-cellular matrix network.

A preferred therapeutic use is for skin wound healing.

A further preferred therapeutic use is the treatment of one or more symptoms of a collagen disorder, e.g. of EDS, particularly of classical EDS.

Further preferred features of the *D. biflorus* extract are described in the subsequent section "Preparation of the *D. biflorus* Extract".

### Preparation of the Dolichos biflorus Extract

The *Dolichos biflorus* extract according to the invention may be prepared by known methods of extracting plants or parts thereof. Suitable extraction processes, such as maceration, re-maceration, agitation maceration, digestion, vortex extraction, ultrasonic extraction, countercurrent extraction, percolation, re-percolation, evacolation (extraction under reduced pressure), diacolation, continuous solid/liquid extraction, can be found for example in Hagers Handbuch der pharmazeutischen Praxis (5th Edition, Vol. 2, pp 1029-1030, Springer-Verlag, Berlin-Heidelberg-New York, 1991**).** Extraction can also be conducted under subcritical or supercritical conditions, using as solvents e.g. carbon dioxide, water with or without alcohol or glycols.

In general, the extraction process entails an extraction step, i.e. contacting solid plant material with a solvent with adequate mixing and for a period of time sufficient to ensure adequate exposure of the solid plant material to the solvent. Preferably, said period of time is sufficient to ensure that compounds conferring the desired properties (particularly collagen production enhancing and/or metabolism enhancing properties), especially collagen production enhancing compounds in the plant material can be taken up by the solvent.

The solvent for the extraction of the *Dolichos biflorus* plant or plant parts may be any solvent suitable for extraction of plants or plant parts and is preferably selected from the group consisting of aqueous solvents (such as water or an aqueous buffer) and organic apolar or polar solvents such as low molecular weight alcohols, e.g. from the group consisting of methanol, ethanol, propylene glycol, butylene glycol, pentylene glycol, pentane, hexane, heptane, acetone, ethyl methyl ketone, ethyl acetate, mixtures thereof and water-containing mixtures thereof, more preferably from the group consisting of water and organic polar solvents such as methanol, ethanol, butylene glycol, mixtures thereof and water-containing mixtures thereof. Even more preferably, the solvent is selected from the group consisting of mixtures of an aqueous solvent, especially water, with one or more polar organic solvent, preferably with a water-miscible organic solvent, more preferably with a low molecular weight alcohol. A mixture of water with an organic solvent selected from the group consisting of 1,3-butyleneglycol, methanol, ethanol, and mixtures thereof, particularly one of the mixtures described in the Examples section, is especially preferred.

In a preferred aspect, the extract is obtained by alcoholic or hydroalcoholic extraction, particularly by extraction with a solvent selected from the group consisting of water, methanol, ethanol and water-containing mixtures thereof, specifically water/ethanol and water/methanol mixtures, more specifically water/ethanol mixtures.

When the extraction process is carried out using a water-containing mixture, i.e. a mixture of an aqueous solvent with an organic solvent, the content of the organic solvent in the mixture ranges from 5% to 95% by volume, preferably from 40 to 90% (v/v). A content of organic solvent from 60 to 80% (v/v), especially of 70% (v/v) is particularly preferred.

For uses wherein the extract will be formulated for application to the skin, a solvent that is compatible with said skin may be selected. Examples of such solvents include water, aqueous buffers, a low molecular weight alcohol, and a mixture of an aqueous solvent with a low molecular weight alcohol.

The extraction process is generally carried out at a temperature from 4 to 180°C, preferably from 20 to 150°C, more preferably from 20 to 80°C. Extraction at room temperature or under reflux - i.e. at the boiling temperature of the solvent - is particularly preferred.

The extraction process may be carried out in an inert gas atmosphere to avoid oxidation of the active principles of the extract.

The extraction process is generally performed at a pH from 2.5 to 11, preferably from 4 to 9, more preferably at light acidic or neutral pH, most preferably at about the pH values indicated in the Examples. In a specific preferred aspect, when the solvent is water, the pH is preferably adjusted to an acidic or neutral pH, more preferably a pH from 4 to 8, even more preferably from 4.5 to 7.5. The adjustment may be achieved by addition of any acid or base which is suitable to achieve the required pH, but addition of pH adjusting agents which are pharmaceutically acceptable ingredients is preferred. In one aspect, the pH is lowered by addition of sulfuric acid.

The plant material may be pretreated prior to the extraction process in order to facilitate the extraction process. Typically, this pretreatment results in plant material being fragmented, such that a greater surface area is presented to the solvent. For example, the plant material can be crushed or sliced mechanically, using a grinder or other fragmenting device.

The particle size of the raw material and the extraction times are routinely selected by the expert in dependence upon the extraction process, the solvent, the temperature and pH conditions, and the ratio of solvent to raw material, etc. Generally, an extraction time of from 40 min to 20 h, preferably of about 1 to 2 h is sufficient. The extraction process may be carried out to any degree, but is usually continued to exhaustion.

During or after the extraction process, the crude extracts may be subjected to other typical purification and workup steps, such as for example purification, clarification, concentration, decoloration, and/or enzymatic hydrolysis. Preferably, such steps take place after the extraction step.

In one preferred aspect, an enzymatic hydrolysis is performed during or after the extraction process. In said aspect, a hydrolytic enzyme is added to the extraction mixture. Said enzyme is preferably a protease, preferably a protease used in the food or feed industry, most preferably alcalase^{®} or a protease with similar enzymatic properties. When an enzyme is added, the preferred solvent is water or an aqueous mixture. Preferably, the pH is at or about the pH optimum of the protease. Generally, the pH is a pH from 4 to 9, preferably from 6 to 8.5, most preferably a pH at about 7.5.

If desired, the extract thus prepared may be subjected to further purification steps, using membrane separation techniques such as nanofiltration, ultrafiltration, precipitation techniques, adsorption/desorption techniques on resins, and/or chromatography techniques.

If desired, the extract may be subjected, for example, to the selective removal of individual unwanted ingredients. Alternatively, an extraction method is selected whereby unwanted ingredients are extracted from the plant material only in a reduced amount or not at all, e.g. by using a hydroalcoholic or alcoholic extraction solvent. In a preferred aspect, the extraction of LEA proteins from the plant material is reduced or avoided, e.g. by choice of an appropriate extraction method, extraction solvent (e.g. alcoholic or hydroalcoholic) and/or raw material (e.g. *D. biflorus* seeds), or LEA are removed from the crude extract by one or more further method steps. Most preferably, the resulting extract is virtually free of LEA.

If desired, the extract may be subjected to drying processes, for example to spray drying or freeze drying. In the context of present invention, the *D. biflorus* extract is preferably dried before its use in skin treatment.

Typical yields of dry matter extracted from the raw plant material based on the quantity of raw material used are in the range from 0.5 to 40% by weight (w/w) and more particularly in the range from 2 to 20% (w/w). The extraction conditions and the yields of the final extracts may be selected by the expert according to the desired application.

The extract may be mixed with one or more auxiliaries, such as e.g. mannitol, sorbitol, maltodextrine, cyclodextrine, glycerine, sugars as saccharose, fructose, glucose and trehalose.

Some of the *D. biflorus* extracts prepared by the method according to present invention are novel. These novel extracts are considered in the following. In one aspect, the extract is an alcoholic or hydroalcoholic extract, preferably a hydroalcoholic extract. In another aspect, the extract is made from *D*. *biflorus* seeds, preferably from dried *D. biflorus* seeds. Particularly preferred is a hydroalcoholic extract of dried seeds, especially a hydroalcoholic extract prepared with one of the preferred solvents as outlined above.

### Pharmaceutical Composition Comprising D. biflorus Extract

The invention is further directed to a pharmaceutical composition comprising a *D*. *biflorus* extract, particularly a *D. biflorus* extract as described above.

The pharmaceutical composition comprises the *D. biflorus* extract in a therapeutically effective amount, i.e. in such an amount that the *D. biflorus* extract achieves the desired therapeutical effect when applied to a patient.

Said composition preferably contains the *D. biflorus* extract in a concentration from about 0.0001 to about 10% and more preferentially from about 0.001 to about 5%, even more preferentially from about 0.01 tp about 2% by weight based on the total composition weight.

In a particular aspect, said composition may comprise one or more additional pharmaceutically active ingredients besides the *D. biflorus* extract.

In a preferred aspect, said composition comprises the *D. biflorus* extract as the only therapeutically active ingredient.

In a further aspect, said composition comprises one or more additional collagen production enhancing ingredients.

In a preferred aspect, said composition comprises the *D. biflorus* extract as the only collagen I, III, IV, V, VI, VII, XII, XIV, XVI, XVIII, and/or XIX production enhancing ingredient, more preferably as the only collagen I and/or V production enhancing ingredient, even more preferably as the only collagen I production enhancing ingredient, and most preferably as the only collagen production enhancing ingredient.

In a further preferred aspect, said composition comprises the *D. biflorus* extract as the only metabolism enhacer, antioxidant, antinflammatory agent and/or agent against oxidative stress.

The composition according to the invention is preferably used in therapeutic skin treatment. One preferred use of the composition is for enhancement of collagen production, especially of collagen I, III, IV, V, VI, VII, XII, XIV, XVI, XVIII, and/or XIX production, particularly of collagen I and/or V production. Another preferred use is the use for metabolism enhancement.

The composition according to the invention is a pharmaceutical composition. For the distinction between cosmetic and pharmaceutical compositions and their corresponding use, reference is made to the legal provisions of the German Federal Republic of Germany (e.g. Kosmetikverordnung, Lebensmittel- und Arzneimittelgesetz).

A preferred pharmaceutical composition in the context of present invention is a topical composition, especially a composition intended to be placed into contact with the skin. Said composition may be used for skin protection, and/or skin regeneration. The effects of the pharmaceutical composition preferably encompass the therapeutic effects described below in the section "Use of the *D. biflorus* extract and composition".

The composition according to present invention can take the form of solutions, suspensions, emulsions, pellets, multiparticulates, powders, sustained release formulations, sprays, or any other form suitable for topical use. The composition according to present invention may be a cream, gel, lotion, paste, foam, an aqueous, alcoholic or aqueous/alcoholic solution, suspension, emulsion, milk, wax/fat mass, stick preparation, powder or ointment. Liquid preparations (like solutions) and semisolid preparations (like creams) as defined in the Ph. Eur., 6th ed., are preferred.

The composition may also comprise one or more further pharmaceutically acceptable ingredients, e.g. a carrier, excipient, auxiliary and/or additive, such as mild surfactants, oil bodies, emulsifiers, consistency regulators, thickeners, superfatting agents, stabilizers, polymers, silicone compounds, fats, waxes, lecithins, phospholipids, UV photoprotective factors, biogenic active ingredients, antioxidants, film formers, swelling agents, hydrotropes, solubilizers, preservatives and the like.

Typically, the composition comprises the *D. biflorus* extract and a pharmaceutically acceptable excipient.

Such pharmaceutical excipients can be liquids, such as water and oils, including those of petroleum, animal, vegetable, or synthetic origin, such as peanut oil, soybean oil, mineral oil, and sesame oil. The pharmaceutical excipients can be saline, gum acacia, gelatin, starch paste, talc, keratin, colloidal silica, and urea. In one embodiment, the pharmaceutically acceptable excipients are sterile when administered to an animal. The solvents described as preferred extraction solvents above are particularly useful as liquid excipients. Saline solutions and aqueous dextrose and glycerol solutions can also be employed as liquid excipients. Other examples of suitable pharmaceutical excipients are described in Remington's Pharmaceutical Sciences pp. 1447-1676 (Alfonso R. Gennaro ed., 19th ed. 1995**).**

Various delivery systems are known for topical administration, e.g., encapsulation in liposomes, and can be used in the pharmaceutical composition comprising the *D*. *biflorus* extract.

A composition according to present invention is typically prepared by a method comprising admixing a *D. biflorus* extract and a pharmaceutically acceptable excipient. Admixing can be accomplished using known methods.

Basic excipients and carriers for pharmaceutical compositions and suitable ingredients therefore are well known and documented in several textbooks, e.g. in Schrader, Grundlagen und Rezepturen der Kosmetika, Huthig Verlag, Heidelberg, 1989**,** Umbach, Kosmetik: Entwicklung, Herstellung und Anwendung kosmetischer Mittel, 2. erweiterte Auflage, 1995, Georg Thieme Verlag**,** or Bauer, Frömming, Fuhrer, Lehrbuch der pharmazeutischen Technologie, 7. Auflage, Wissenschaftliche Verlagsgesellschaft mbH Stuttgart, 2002**.**

In one preferred aspect of present invention the composition further comprises at least one surfactant.

Surface-active substances which may be present are anionic, nonionic, cationic and/or amphoteric or zwitterionic surfactants, the content of which in the compositions is usually about 1 to 70% by weight, preferably 5 to 50% by weight and in particular 10 to 30% by weight. Typical examples of anionic surfactants are soaps, alkylbenzenesulphonates, alkanesulphonates, olefinsulphonates, alkyl ether sulphonates, glycerol ether sulphonates, α-methyl ester sulphonates, sulpho fatty acids, alkyl sulphates, alkyl ether sulphates, glycerol ether sulphates, fatty acid ether sulphates, hydroxy mixed ether sulphates, monoglyceride (ether) sulphates, fatty acid amide (ether) sulphates, mono- and dialkyl sulphosuccinates, mono- and dialkyl sulphosuccinamates, sulphotriglycerides, amide soaps, ether carboxylic acids and salts thereof, fatty acid isethionates, fatty acid sarcosinates, fatty acid taurides, N-acylaminoacids, such as, for example, acyl lactylates, acyl tartrates, acyl glutamates and acyl aspartates, alkyl oligoglucoside sulphates, protein fatty acid condensates (in particular wheat-based vegetable products) and alkyl (ether) phosphates. If the anionic surfactants comprise polyglycol ether chains, these can have a conventional homologue distribution, but preferably have a narrowed homologue distribution. Typical examples of nonionic surfactants are fatty alcohol polyglycol ethers, alkylphenol polyglycol ethers, fatty acid polyglycol esters, fatty acid amide polyglycol ethers, fatty amine polyglycol ethers, alkoxylated triglycerides, mixed ethers and mixed formals, optionally partially oxidized alk(en)yl oligoglycosides and glucoronic acid derivatives, fatty acid N-alkylglucamides, protein hydrolysates (in particular wheat-based vegetable products), polyol fatty acid esters, sugar esters, sorbitan esters, polysorbates and amine oxides. If the nonionic surfactants contain polyglycol ether chains, these can have a conventional homologue distribution, but preferably have a narrowed homologue distribution. Typical examples of cationic surfactants are quaternary ammonium compounds, such as, for example, dimethyldistearyl-ammonium chloride, and ester quats, in particular quaternized fatty acid trialkanolamine ester salts. Typical examples of amphoteric and zwitterionic surfactants are alkylbetaines, alkylamidobetaines, aminopropionates, aminoglycinates, imidazoliniumbetaines and sulphobetaines. The specified surfactants are exclusively known compounds, whose preparation and structure are known from textbooks like J. Falbe (ed.), "Surfactants in Consumer Products", Springer Verlag Berlin, 1987, pp 54-124**, or** J. Falbe (ed.), "Katalysatoren, Tenside und Mineralöladditive", Thieme Verlag Stuttgart, 1978, pp 123-217**.** Typical examples of particularly suitable mild, i.e. particularly skin-compatible, surfactants are fatty alcohol polyglycol ether sulphates, monoglyceride sulphates, mono- and/or dialkyl sulphosuccinates, fatty acid isethionates, fatty acid sarcosinates, fatty acid taurides, fatty acid glutamates, α-olefinsulphonates, ether carboxylic acids, alkyl oligoglucosides, fatty acid glucamides, alkylamidobetaines, amphoacetals and/or protein fatty acid condensates, the latter preferably being based on wheat proteins.

In a further preferred aspect of the invention the composition further comprises at least one oil body.

Suitable oil bodies are, for example, Guerbet alcohols based on fatty alcohols having 6 to 18, preferably 8 to 10, carbon atoms, esters of linear C₆-C₂₂-fatty acids with linear or branched C₆-C₂₂-fatty alcohols and/or esters of branched C₆-C₁₃-carboxylic acids with linear or branched C₆-C₂₂-fatty alcohols, such as, for example, myristyl myristate, myristyl palmitate, myristyl stearate, myristyl isostearate, myristyl oleate, myristyl behenate, myristyl erucate, cetyl myristate, cetyl palmitate, cetyl stearate, cetyl isostearate, cetyl oleate, cetyl behenate, cetyl erucate, stearyl myristate, stearyl palmitate, stearyl stearate, stearyl isostearate, stearyl oleate, stearyl behenate, stearyl erucate, isostearyl myristate, isostearyl palmitate, isostearyl stearate, isostearyl isostearate, isostearyl oleate, isostearyl behenate, isostearyl oleate, oleyl myristate, oleyl palmitate, oleyl stearate, oleyl isostearate, oleyl oleate, oleyl behenate, oleyl erucate, behenyl myristate, behenyl palmitate, behenyl stearate, behenyl isostearate, behenyl oleate, behenyl behenate, behenyl erucate, erucyl myristate, erucyl palmitate, erucyl stearate, erucyl isostearate, erucyl oleate, erucyl behenate and erucyl erucate. Also suitable are esters of linear C₆-C₂₂-fatty acids with branched alcohols, in particular 2-ethylhexanol, esters of C₁₈-C₃₈-alkyl hydroxycarboxylic acids with linear or branched C₆-C₂₂-fatty alcohols (like, e.g., the ones described in DE 19756377 A1), in particular dioctyl malates, esters of linear and/or branched fatty acids with polyhydric alcohols (such as, for example, propylene glycol, dimerdiol or trimertriol) and/or Guerbet alcohols, triglycerides based on C₆-C₁₀-fatty acids, liquid mono-/di-/triglyceride mixtures based on C₆-C₁₈-fatty acids, esters of C₆-C₂₂-fatty alcohols and/or Guerbet alcohols with aromatic carboxylic acids, in particular benzoic acid, esters of C₂-C₁₂-dicarboxylic acids with linear or branched alcohols having 1 to 22 carbon atoms or polyols having 2 to 10 carbon atoms and 2 to 6 hydroxyl groups, vegetable oils, branched primary alcohols, substituted cyclohexanes, linear and branched C₆-C₂₂-fatty alcohol carbonates, such as, for example, dicaprylyl carbonate (Cetiol^{®} CC), Guerbet carbonates based on fatty alcohols having 6 to 18, preferably 8 to 10, carbon atoms, esters of benzoic acid with linear and/or branched C₆-C₂₂-alcohols (e.g. Finsolv^{®} TN), linear or branched, symmetrical or unsymmetrical dialkyl ethers having 6 to 22 carbon atoms per alkyl group, such as, for example, dicaprylyl ether (Cetiol^{®} OE), ring-opening products of epoxidized fatty acid esters with polyols, silicone oils (cyclomethicones, silicon methicone types, inter alia) and/or aliphatic or naphthenic hydrocarbons, such as, for example, squalane, squalene or dialkylcyclohexanes.

In a further preferred aspect of the invention the composition further comprises at least one emulsifier.

Suitable emulsifiers are, for example, nonionogenic surfactants selected from at least one of the following groups:
- addition products of from 2 to 30 mol of ethylene oxide and/or 0 to 5 mol of propylene oxide to linear fatty alcohols having 8 to 22 carbon atoms, to fatty acids having 12 to 22 carbon atoms, to alkylphenols having 8 to 15 carbon atoms in the alkyl group, and alkylamines having 8 to 22 carbon atoms in the alkyl radical;
- alkyl and/or alkenyl oligoglycosides having 8 to 22 carbon atoms in the alk(en)yl radical and the ethoxylated analogues thereof;
- addition products of from 1 to 15 mol of ethylene oxide to castor oil and/or hydrogenated castor oil;
- addition products of from 15 to 60 mol of ethylene oxide to castor oil and/or hydrogenated castor oil;
- partial esters of glycerol and/or sorbitan with unsaturated, linear or saturated, branched fatty acids having 12 to 22 carbon atoms and/or hydroxycarboxylic acids having 3 to 18 carbon atoms, and the adducts thereof with 1 to 30 mol of ethylene oxide;
- partial esters of polyglycerol (average degree of self-condensation 2 to 8), polyethylene glycol (molecular weight 400 to 5000), trimethylolpropane, pentaerythritol, sugar alcohols (e.g. sorbitol), alkyl glucosides (e.g. methyl glucoside, butyl glucoside, lauryl glucoside), and polyglucosides (e.g. cellulose) with saturated and/or unsaturated, linear or branched fatty acids having 12 to 22 carbon atoms and/or hydroxycarboxylic acids having 3 to 18 carbon atoms, and the adducts thereof with 1 to 30 mol of ethylene oxide;
- mixed esters of pentaerythritol, fatty acids, citric acid and fatty alcohol (like, e.g., the ones described in DE 1165574) and/or mixed esters of fatty acids having 6 to 22 carbon atoms, methylglucose and polyols, preferably glycerol or polyglycerol,
- mono-, di- and trialkyl phosphates, and mono-, di- and/or tri-PEG alkyl phosphates and salts thereof;
- wool wax alcohols;
- polysiloxane-polyalkyl-polyether copolymers and corresponding derivatives;
- block copolymers, e.g. polyethylene glycol-30 dipolyhydroxystearates;
- polymer emulsifiers, e.g. Pemulen grades (TR-1, TR-2) from Goodrich;
- polyalkylene glycols, and
- glycerol carbonate.

Some of these groups are more particularly defined as follows:

### • Ethylene oxide addition products

The addition products of ethylene oxide and/or of propylene oxide to fatty alcohols, fatty acids, alkylphenols or to castor oil are known, commercially available products. These are homologue mixtures whose average degree of alkoxylation corresponds to the ratio of the amounts of substance of ethylene oxide and/or propylene oxide and substrate with which the addition reaction is carried out. C_{12/18}-fatty acid mono- and diesters of addition products of ethylene oxide to glycerol are known as retailing agents (e.g. from DE 2024051).

### • Alkyl and/or alkenyl oligoglycosides

Alkyl and/or alkenyl oligoglycosides, their preparation and their use are known from the prior art. They are prepared, in particular, by reacting glucose or oligosaccharides with primary alcohols having 8 to 18 carbon atoms. With regard to the glycoside radical, both monoglycosides, in which a cyclic sugar radical is glycosidically bonded to the fatty alcohol, and also oligomeric glycosides having a degree of oligomerization of up to, preferably, about 8, are suitable. The degree of oligomerization here is a statistical average value which is based on a homologue distribution customary for such technical-grade products.

### • Partial glycerides

Typical examples of suitable partial glycerides are hydroxystearic acid monoglyceride, hydroxystearic acid diglyceride, isostearic acid monoglyceride, isostearic acid diglyceride, oleic acid monoglyceride, oleic acid diglyceride, ricinoleic acid monoglyceride, ricinoleic acid diglyceride, linoleic acid monoglyceride, linoleic acid diglyceride, linolenic acid monoglyceride, linolenic acid diglyceride, erucic acid monoglyceride, erucic acid diglyceride, tartaric acid monoglyceride, tartaric acid diglyceride, citric acid monoglyceride, citric acid diglyceride, malic acid monoglyceride, malic acid diglyceride, and the technical-grade mixtures thereof which may also comprise small amounts of triglyceride as a minor product of the preparation process. Likewise suitable are addition products of 1 to 30 mol, preferably 5 to 10 mol, of ethylene oxide to said partial glycerides.

### • Sorbitan esters

Suitable sorbitan esters are sorbitan monoisostearate, sorbitan sesquiisostearate, sorbitan diisostearate, sorbitan triisostearate, sorbitan monooleate, sorbitan sesquioleate, sorbitan dioleate, sorbitan trioleate, sorbitan monoerucate, sorbitan sesquierucate, sorbitan dierucate, sorbitan trierucate, sorbitan monoricinoleate, sorbitan sesquiricinoleate, sorbitan diricinoleate, sorbitan triricinoleate, sorbitan monohydroxystearate, sorbitan sesquihydroxystearate, sorbitan dihydroxystearate, sorbitan trihydroxystearate, sorbitan monotartrate, sorbitan sesquitartrate, sorbitan ditartrate, sorbitan tritartrate, sorbitan monocitrate, sorbitan sesquicitrate, sorbitan dicitrate, sorbitan tricitrate, sorbitan monomaleate, sorbitan sesquimaleate, sorbitan dimaleate, sorbitan trimaleate, and technical-grade mixtures thereof. Likewise suitable are addition products of from 1 to 30 mol, preferably from 5 to 10 mol, of ethylene oxide to said sorbitan esters.

### • Polyglycerol esters

Typical examples of suitable polyglycerol esters are polyglyceryl-2 dipolyhydroxystearate (Dehymuls^{®} PGPH), polyglycerol-3 diisostearate (Lameform^{®} TGI), polyglyceryl-4 isostearate (Isolan^{®} GI 34), polyglyceryl-3 oleate, diisostearoyl polyglyceryl-3 diisostearate (Isolan^{®} PDI), polyglyceryl-3 methylglucose distearate (Tego Care^{®} 450), polyglyceryl-3 beeswax (Cera Beiiina^{®}), polyglyceryl-4 caprate (Polyglycerol Caprate T2010/90), polyglyceryl-3 cetyl ether (Chimexane^{®} NL), polyglyceryl-3 distearate (Cremophor^{®} GS 32) and polyglyceryl polyricinoleate (Admul^{®} WOL 1403), polyglyceryl dimerate isostearate, and mixtures thereof. Examples of further suitable polyol esters are the mono-, di- and triesters, optionally reacted with 1 to 30 mol of ethylene oxide, of trimethylolpropane or pentaerythritol with lauric acid, coconut fatty acid, tallow fatty acid, palmitic acid, stearic acid, oleic acid, behenic acid and the like.

Further suitable emulsifiers are, for example, anionic, amphoteric or cationic emulsifiers selected from at least one of the following groups:

### • Anionic emulsifiers

Typical anionic emulsifiers are aliphatic fatty acids having 12 to 22 carbon atoms, such as, for example, palmitic acid, stearic acid or behenic acid, and dicarboxylic acids having 12 to 22 carbon atoms, such as, for example, azelaic acid or sebacic acid.

### • Amphoteric and cationic emulsifiers

Furthermore, zwitterionic surfactants can be used as emulsifiers. The term "zwitterionic surfactants" refers to those surface-active compounds which carry at least one quaternary ammonium group and at least one carboxylate and one sulphonate group in the molecule. Particularly suitable zwitterionic surfactants are the so-called betaines, such as N-alkyl-N,N-dimethylammonium glycinates, for example cocoalkyldimethylammonium glycinate, N-acylaminopropyl-N,N-dimethylammonium glycinates, for example cocoacylaminopropyldimethylammonium glycinate, and 2-alkyl-3-carboxymethyl-3-hydroxyethylimidazolines having in each case 8 to 18 carbon atoms in the alkyl or acyl group, and cocoacylaminoethylhydroxyethylcarboxymethyl glycinate. Particular preference is given to the fatty acid amide derivative known under the CTFA name Cocamidopropyl Betaine. Likewise suitable emulsifiers are ampholytic surfactants. The term "ampholytic surfactants" means those surface-active compounds which, apart from a C_{8/18}-alkyl or -acyl group, contain at least one free amino group and at least one -COOH or -SO₃H group in the molecule and are capable of forming internal salts. Examples of suitable ampholytic surfactants are N-alkylglycines, N-alkylaminopropionic acids, N-alkylaminobutyric acids, N-alkyliminodipropionic acids, N-hydroxyethyl-N-alkylamidopropylglycines, N-alkyltaurines, N-alkylsarcosines, 2-alkylaminopropionic acids and alkylaminoacetic acids having in each case about 8 to 18 carbon atoms in the alkyl group. Particularly preferred ampholytic surfactants are N-cocoalkylaminopropionate, cocoacylaminoethylaminopropionate and C_{12/18}-acylsarcosine. Finally, cationic surfactants are also suitable as emulsifiers, those of the ester quat type, preferably methyl-quaternized difatty acid triethanolamine ester salts, being particularly preferred.

In a further preferred aspect of the invention the composition further comprises at least one fat or wax.

Typical examples of fats are glycerides, i.e. solid or liquid vegetable or animal products which consist essentially of mixed glycerol esters of higher fatty acids, suitable waxes are inter alia natural waxes, such as, for example, candelilla wax, carnauba wax, Japan wax, esparto grass wax, cork wax, guaruma wax, rice germ oil wax, sugarcane wax, ouricury wax, montan wax, beeswax, shellac wax, spermaceti, lanolin (wool wax), uropygial grease, ceresin, ozokerite (earth wax), petrolatum, paraffin waxes, microcrystalline waxes; chemically modified waxes (hard waxes), such as, for example, montan ester waxes, sasol waxes, hydrogenated jojoba waxes, and synthetic waxes, such as, for example, polyalkylene waxes and polyethylene glycol waxes. In addition to the fats, suitable additives are also fat-like substances, such as lecithins and phospholipids. The term lecithins is understood by the person skilled in the art as meaning those glycerophospholipids which are founded from fatty acids, glycerol, phosphoric acid and choline by esterification. Lecithins are thus also often designated as phosphatidylcholines (PC) in the specialist world. Examples of natural lecithins which may be mentioned are the cephalins, which are also referred to as phosphatidic acids and constitute derivatives of 1,2-diacyl-sn-glycerol-3-phosphoric acids. By contrast, phospholipids are usually understood as meaning mono- and preferably diesters of phosphoric acid with glycerol (glycerol phosphates), which are generally classed as fats. In addition, sphingosines or sphingolipids are also suitable.

In a further preferred aspect of the invention the composition further comprises at least one consistency regulator and/or thickener.

Suitable consistency regulators are primarily fatty alcohols or hydroxy fatty alcohols having 12 to 22, and preferably 16 to 18, carbon atoms, and also partial glycerides, fatty acids or hydroxy fatty acids. Preference is given to a combination of these substances with alkyl oligoglucosides and/or fatty acid N-methylglucamides of identical chain length and/or polyglycerol poly-12-hydroxystearates. Suitable thickeners are, for example, Aerosil grades (hydrophilic silicas), polysaccharides, in particular xanthan gum, guar guar, agar agar, alginates and tyloses, carboxymethylcellulose, hydroxyethylcellulose and hydroxypropylcellulose, and also relatively high molecular weight polyethylene glycol mono- and diesters of fatty acids, polyacrylates (e.g. Carbopols^{®} and Pemulen grades from Goodrich; Synthalens^{®} from Sigma; Keltrol grades from Kelco; Sepigel grades from Seppic; Salcare grades from Allied Colloids), polyacrylamides, polymers, polyvinyl alcohol and polyvinylpyrrolidone. Bentonites, such as, for example, Bentone^{®} Gel VS 5PC (Rheox), which is a mixture of cyclopentasiloxane, disteardimonium hectorite and propylene carbonate, have also proven to be particularly effective. Also suitable are surfactants, such as, for example, ethoxylated fatty acid glycerides, esters of fatty acids with polyols such as, for example, pentaerythritol or trimethylolpropane, fatty alcohol ethoxylates having a narrowed homologue distribution or alkyl oligoglucosides, and electrolytes such as sodium chloride and ammonium chloride.

In a further preferred aspect of the invention the composition further comprises at least one superfatting agent.

Superfatting agents which can be used are substances such as, for example, lanolin and lecithin, and polyethoxylated or acylated lanolin and lecithin derivatives, polyol fatty acid esters, monoglycerides and fatty acid alkanolamides, the latter also serving as foam stabilizers.

In a further preferred aspect of the invention the composition further comprises at least one stabilizer.

Stabilizers which can be used are metal salts of fatty acids, such as, for example, magnesium, aluminium and/or zinc stearate or ricinoleate.

In a further preferred aspect of the invention the composition further comprises at least one polymer.

Suitable cationic polymers are, for example, cationic cellulose derivatives, such as, for example, a quaternized hydroxyethylcellulose obtainable under the name Polymer JR 400^{®} from Amerchol, cationic starch, copolymers of diallylammonium salts and acrylamides, quaternized vinylpyrrolidone-vinylimidazole polymers, such as, for example, Luviquat^{®} (BASF), condensation products of polyglycols and amines, quaternized collagen polypeptides, such as, for example, lauryldimonium hydroxypropyl hydrolyzed collagen (Lamequat^{®}L/Grunau), quaternized wheat polypeptides, polyethyleneimine, cationic silicone polymers, such as, for example, amodimethicones, copolymers of adipic acid and dimethylaminohydroxypropyldiethylenetriamine (Cartaretins^{®}/Sandoz), copolymers of acrylic acid with dimethyldiallylammonium chloride (Merquat^{®} 550/Chemviron), polyaminopolyamides (like the ones described in FR 2252840), and crosslinked water-soluble polymers thereof, cationic chitin derivatives, such as, for example, quaternized chitosan, optionally in microcrystalline dispersion, condensation products from dihaloalkyls, such as, for example, dibromobutane with bisdialkylamines, such as, for example, bis-dimethylamino-1,3-propane, cationic guar gum, such as, for example, Jaguar^{®} CBS, Jaguar^{®} C-17, Jaguar^{®} C-16 from Celanese, quaternized ammonium salt polymers, such as, for example, Mirapol^{®} A-15, Mirapol^{®} AD-1, Mirapol^{®} AZ-1 from Miranol.

Suitable anionic, zwitterionic, amphoteric and nonionic polymers are, for example, vinyl acetate-crotonic acid copolymers, vinylpyrrolidone-vinyl acrylate copolymers, vinyl acetate-butyl maleate-isobornyl acrylate copolymers, methyl vinyl ether-maleic anhydride copolymers and esters thereof, uncrosslinked polyacrylic acids and polyacrylic acids crosslinked with polyols, acrylamidopropyltrimethylammonium chloride-acrylate copolymers, octylacrylamide-methyl methacrylate-tert-butylaminoethyl methacrylate-2-hydroxypropyl methacrylate copolymers, polyvinylpyrrolidone, vinylpyrrolidone-vinyl acetate copolymers, vinylpyrrolidone-dimethylaminoethyl methacrylate-vinylcaprolactam terpolymers, and optionally derivatized cellulose ethers and silicones. Further suitable polymers and thickening agents are described in Cosm. Toil. (1993) 108:95**.**

In a further preferred aspect of the invention the composition further comprises at least one silicone compound.

Suitable silicone compounds are, for example, dimethylpolysiloxanes, methylphenylpolysiloxanes, cyclic silicones, and amino-, fatty acid-, alcohol-, polyether-, epoxy-, fluorine-, glycoside- and/or alkyl-modified silicone compounds, which can either be liquid or in resin form at room temperature. Also suitable are simethicones, which are mixtures of dimethicones having an average chain length of from 200 to 300 dimethylsiloxane units and hydrogenated silicates. A detailed survey on suitable volatile silicones is provided by Todd et al. (1976) Cosm. Toil. 91:27**.**

In a further aspect of the invention the composition further comprises at least one UV photoprotective factor.

Suitable UV photoprotective factors are, for example, to be understood as comprising organic substances (photoprotective filters) which are liquid or crystalline at room temperature and which are able to absorb ultraviolet rays and give off the absorbed energy again in the form of longer-wavelength radiation, e.g. heat. UV-B filters may be oil-soluble or water-soluble. Examples of oil-soluble UV-B filters are:
- 3-benzylidenecamphor or 3-benzylidenenorcamphor and derivatives thereof (e.g. as described in EP 0693471 B1**)**, e.g. 3-(4-methylbenzylidene)camphor;
- 4-aminobenzoic acid derivatives, preferably 2-ethylhexyl 4-(dimethylamino) benzoate, 2-octyl 4-(dimethylamino)benzoate and amyl 4-(dimethylamino)-benzoate;
- esters of cinnamic acid, preferably 2-ethylhexyl 4-methoxycinnamate, propyl 4-methoxycinnamate, isoamyl 4-methoxycinnamate, 2-ethylhexyl 2-cyano-3,3-phenylcinnamate (octocrylene);
- esters of salicylic acid, preferably 2-ethylhexyl salicylate, 4-isopropylbenzyl salicylate, homomenthyl salicylate;
- derivatives of benzophenone, preferably 2-hydroxy-4-methoxybenzophenone, 2-hydroxy-4-methoxy-4'-methylbenzophenone, 2,2'-dihydroxy-4-methoxybenzophenone;
- esters of benzalmalonic acid, preferably di-2-ethylhexyl-4-methoxybenzalmalonate;
- triazine derivatives, such as, for example, 2,4,6-trianilino(p-carbo-2'-ethyl-1'-hexyloxy)-1,3,5-triazine and octyltriazone (e.g., as described in EP 0818450 A1**)** or dioctylbutamidotriazone (Uvasorb^{®} HEB);
- propane-1,3-diones, such as, for example, 1-(4-tert-butylphenyl)-3-(4'-methoxyphenyl)propane-1,3-dione;
- ketotricyclo(5.2.1.0)decane derivatives (e.g. as described in EP 0694521 B1**).**

Suitable water-soluble UV-B filters are:
- 2-phenylbenzimidazole-5-sulphonic acid and the alkali metal, alkaline earth metal, ammonium, alkylammonium, alkanolammonium and glucammonium salts thereof;
- sulphonic acid derivatives of benzophenones, preferably 2-hydroxy-4-methoxybenzophenone-5-sulphonic acid and its salts;
- sulphonic acid derivatives of 3-benzylidenecamphor, such as, for example, 4-(2-oxo-3-bornylidenemethyl)benzenesulphonic acid and 2-methyl-5-(2-oxo-3-bornylidene)sulphonic acid and salts thereof.

Suitable UV-A filters are, in particular, derivatives of benzoylmethane, such as, for example, 1-(4'-tert-butylphenyl)-3-(4'-methoxyphenyl)propane-1,3-dione, 4-tert-butyl-4'-methoxydibenzoylmethane (Parsol^{®} 1789), 1-phenyl-3-(4'-isopropylphenyl)-propane-1,3-dione, and enamine compounds (e.g as described in DE 19712033 A1**)**. The UV-A and UV-B filters can of course also be used in mixtures. Particularly favourable combinations consist of the derivatives of benzoylmethane, e.g. 4-tert-butyl-4'-methoxydi-benzoylmethane (Parsol^{®} 1789) and 2-ethylhexyl 2-cyano-3,3-phenylcinnamate (octocrylene) in combination with esters of cinnamic acid, preferably 2-ethylhexyl 4-methoxycinnamate and/or propyl 4-methoxycinnamate and/or isoamyl 4-methoxycinnamate. Advantageously, such combinations are combined with water-soluble filters such as, for example, 2-phenylbenzimidazole-5-sulphonic acid and their alkali metal, alkaline earth metal, ammonium, alkylammonium, alkanolammonium and glucammonium salts.

As well as said soluble UV protective factors, insoluble light protection pigments, namely finely dispersed metal oxides or salts, are also suitable for this purpose. Examples of suitable metal oxides are, in particular, zinc oxide and titanium dioxide and also oxides of iron, zirconium, silicon, manganese, aluminium and cerium, and mixtures thereof. Salts which may be used are silicates (talc), barium sulphate or zinc stearate. The oxides and salts are used in the form of the pigments for skin-protective emulsions. The particles here should have an average diameter of less than 100 nm, preferably between 5 and 50 nm and in particular between 15 and 30 nm. They can have a spherical shape, but it is also possible to use particles which have an ellipsoidal shape or a shape deviating in some other way from the spherical form. The pigments can also be surface-treated, i.e. hydrophilicized or hydrophobicized. Typical examples are coated titanium dioxides, such as, for example, titanium dioxide T 805 (Degussa) or Eusolex^{®} T2000 (Merck). Suitable hydrophobic coating agents are here primarily silicones and, specifically in this case, trialkoxyoctylsilanes or simethicones. Preference is given to using so-called micro- or nanopigments. Preference is given to using micronized zinc oxide. Further suitable UV protective factors are listed in a survey by P. Finkel in SÖFW-Journal (1996) 122:543 **and** Parf. Cosm. (1999) 3:11**.**

In a further preferred aspect of the invention the composition further comprises at least one biogenic active ingredient and/or antioxidant.

Biogenic active ingredients are understood as meaning, for example, tocopherol, tocopherol acetate, tocopherol palmitate, ascorbic acid, (deoxy)ribonucleic acid and fragmentation products thereof, β-glucans, retinol, bisabolol, allantoin, phytantriol, panthenol, AHA acids, amino acids, ceramides, pseudoceramides, essential oils, additional plant extracts, such as, for example, prunus extract, bambara nut extract and vitamin complexes.

Antioxidants interrupt the photochemical reaction chain which is triggered when UV radiation penetrates the skin. Typical examples thereof are amino acids (e.g. glycine, histidine, tyrosine, tryptophan) and derivatives thereof, imidazoles (e.g. urocanic acid) and derivatives thereof, peptides, such as D,L-carnosine, D-carnosine, L-carnosine and derivatives thereof (e.g. anserine), carotenoids, carotenes (e.g. α-carotene, β-carotene, lycopene) and derivatives thereof, chlorogenic acid and derivatives thereof, lipoic acid and derivatives thereof (e.g. dihydrolipoic acid), aurothioglucose, propylthiouracil and other thiols (e.g. thioredoxin, glutathione, cysteine, cystine, cystamine and the glycosyl, N-acetyl, methyl, ethyl, propyl, amyl, butyl and lauryl, palmitoyl, oleyl, γ-linoleyl, cholesteryl and glyceryl esters thereof) and salts thereof, dilauryl thiodipropionate, distearyl thiodipropionate, thiodipropionic acid and derivatives thereof (esters, ethers, peptides, lipids, nucleotides, nucleosides and salts), and sulphoximine compounds (e.g. buthionine sulphoximines, homocysteine sulphoximine, buthionine sulphones, penta-, hexa-, heptathionine sulphoximine) in very low tolerated doses (e.g. pmol to µmol/kg), and also (metal) chelating agents (e.g. α-hydroxy fatty acids, palmitic acid, phytic acid, lactoferrin), α-hydroxy acids (e.g. citric acid, lactic acid, malic acid), humic acid, bile acid, bile extracts, bilirubin, biliverdin, EDTA, EGTA and derivatives thereof, unsaturated fatty acids and derivatives thereof (e.g. γ-linolenic acid, linoleic acid, oleic acid), folic acid and derivatives thereof, ubiquinone and ubiquinol and derivatives thereof, vitamin C and derivatives (e.g. ascorbyl palmitate, Mg ascorbyl phosphate, ascorbyl acetate), tocopherols and derivatives (e.g. vitamin E acetate), vitamin A and derivatives (vitamin A palmitate), and coniferyl benzoate of gum benzoin, rutic acid and derivatives thereof, α-glycosylrutin, ferulic acid, furfurylideneglucitol, carnosine, butylhydroxytoluene, butylhydroxyanisole, nordihydroguaiacic acid, nordihydroguaiaretic acid, trihydroxybutyrophenone, uric acid and derivatives thereof, mannose and derivatives thereof, superoxide dismutase, zinc and derivatives thereof (e.g. ZnO, ZnSO₄) selenium and derivatives thereof (e.g. selenomethionine), stilbenes and derivatives thereof (e.g. stilbene oxide, trans-stilbene oxide) and the derivatives (salts, esters, ethers, sugars, nucleotides, nucleosides, peptides and lipids) of said active ingredients which are suitable according to the invention.

In a further preferred aspect of the invention the composition further comprises at least one anti-microbial agent and/or preservative.

Suitable antimicrobial agents are, in principle, all substances effective against gram-positive bacteria, such as, for example, 4-hydroxybenzoic acid and its salts and esters, N-(4-chlorophenyl)-N'-(3,4-dichlorophenyl)urea, 2,4,4'-trichloro-2'-hydroxydiphenyl ether (triclosan), 4-chloro-3,5-dimethylphenol, 2,2'-methylenebis(6-bromo-4-chlorophenol), 3-methyl-4-(1-methylethyl)phenol, 2-benzyl-4-chlorophenol, 3-(4-chlorophenoxy)-1,2-propanediol, 3-iodo-2-propynyl butylcarbamate, chlorhexidine, 3,4,4'-trichlorocarbanilide (TTC), antibacterial fragrances, thymol, thyme oil, eugenol, oil of cloves, menthol, mint oil, farnesol, phenoxyethanol, glycerol monocaprate, glycerol monocaprylate, glycerol monolaurate (GML), diglycerol monocaprate (DMC), salicylic acid N-alkylamides, such as, for example, N-octylsalicylamide or N-decylsalicylamide.

Suitable preservatives are, for example, phenoxy ethanol, formaldehyde solution, parabens, pentanediol or sorbic acid, and the silver complexes known under the name Surfacins^{®}, and also the other classes of substance listed in Annex 6, Part A and B of the Cosmetics Directive.

In a further preferred aspect of the invention the composition further comprises at least one film former.

Customary film formers are, for example, chitosan, microcrystalline chitosan, quaternized chitosan, polyvinylpyrrolidone, vinylpyrrolidone-vinyl acetate copolymers, polymers of the acrylic acid series, quaternary cellulose derivatives, collagen, hyaluronic acid and salts thereof, and similar compounds.

In a further preferred aspect of the invention the composition further comprises at least one swelling agent.

The swelling agents for aqueous phases may be montmorillonites, clay mineral substances, Pemulen, and alkyl-modified Carbopol grades (Goodrich). Other suitable polymers and swelling agents are given in a review by R. Lochhead in Cosm. Toil. (1993) 108:95**.**

In a further preferred aspect of the invention the composition further comprises at least one hydrotrophic agent.

To improve the flow behaviour, it is possible to use hydrotropic agents, such as, for example, ethanol, isopropyl alcohol, or polyols. Polyols which are suitable preferably have 2 to 15 carbon atoms and at least two hydroxyl groups. The polyols may also contain further functional groups, in particular amino groups, or be modified with nitrogen. Typical examples are
- glycerol;
- alkylene glycols, such as, for example, ethylene glycol, diethylene glycol, propylene glycol, butylene glycol, hexylene glycol, and polyethylene glycols with an average molecular weight of from 100 to 1 000 daltons;
- technical-grade oligoglycerol mixtures with a degree of self-condensation of from 1.5 to 10, such as, for example, technical-grade diglycerol mixtures with a diglycerol content of from 40 to 50% by weight;
- methylol compounds, such as, in particular, trimethylolethane, trimethylolpropane, trimethylolbutane, pentaerythritol and dipentaerythritol;
- lower alkyl glucosides, in particular those having 1 to 8 carbon atoms in the alkyl radical, such as, for example, methyl and butyl glucoside;
- sugar alcohols having 5 to 12 carbon atoms, such as, for example, sorbitol or mannitol,
- sugars having 5 to 12 carbon atoms, such as, for example, glucose or sucrose;
- amino sugars, such as, for example, glucamine;
- dialcohol amines, such as diethanolamine or 2-amino-1,3-propanediol.

Preferred further components of the composition are skin protective components, especially components selected from the group consisting of UV filters, hydrotrophic agents (especially glycerol), biogenic ingredients and antioxidants.

The total amount of further components besides the *Dolichos biflorus* extract and the optional carrier or excipient in the composition may be from 0 to 90% by weight, and is typically from 1 to 50%, often 5 to 40% by weight, based on the total composition weight. The composition may be prepared by customary cold or hot processes; preference is given to using the phase-inversion temperature method.

### Use of the Dolichos biflorus Extract and Composition

The invention relates to the therapeutic use of a *Dolichos biflorus* extract or of a composition comprising said extract. It also relates to a *Dolichos biflorus* extract for use as a medicament and to a pharmaceutical composition comprising a *Dolichos biflorus* extract for use as a medicament. For the distinction between cosmetic and therapeutic use, reference is made to the legal provisions of the German Federal Republic of Germany (e.g. Kosmetikverordnung, Lebensmittel- und Arzneimittelgesetz).

Due to its activity, the *D. biflorus* extract according to present invention is useful in veterinary and human medicine, human medicine being preferred.

The invention relates to the use of a *D. biflorus* extract as a medicament, i.e. its therapeutic use. In one aspect of present invention, the *D. biflorus* extract is used in the treatment or prevention of a disease caused by impaired or reduced collagen production, by impaired or reduced cell metabolism, by oxidative stress and/or by inflammation, especially of a disease which affects the health, integrity and/or strength of the skin. In a further aspect of present invention, the *D. biflorus* extract is used in restoring or improving the health, integrity and/or strength of collagen containing tissue, especially of the skin, e.g. as a protective measure or after mechanical wounding or burning.

The invention further provides a pharmaceutical composition comprising a *D. biflorus* extract for said therapeutic use.

The invention also relates to the use of a *D. biflorus* extract for the preparation of a medicament for said therapeutic use.

The invention also relates to a process for preventing and treating the conditions and diseases listed herein, which process comprises administering a therapeutically effective amount of the *D. biflorus* extract according to present invention to a patient in need thereof. The patient may be animal or human, and is preferably a mammal, more preferably a human.

*A Dolichos biflorus* extract stimulates the mRNA production and production of the collagen proteins collagen I and V of cultured human fibroblasts (compare Examples 9 and 10). It also stimulates the metabolism of cultured human fibroblasts (compare Example 8), especially their energy metabolism (e.g. their ATP synthesis rate).

In addition to its effects on fibroblasts metabolism and production of collagen I and V, a *Dolichos biflorus* extract has demonstrated a protective effect against oxidative damages on fibroblasts and keratinocytes in culture (compare Examples 11 and 12).

In one embodiment, the *D. biflorus* extract is for use as a medicament for treatment or prevention of a disease caused by impaired or reduced collagen production. Examples of such diseases are collagen disorders, scurvy and collagenopathy. Collagen disorders are preferably the different kinds of EDS, especially classical EDS. Scurvy is a disease resulting from a deficiency of vitamin C, which is required for the synthesis of collagen in humans. As demonstrated in the Examples, a *D*. *biflorus* extract according to present invention has similar effects as vitamin C on collagen production. Collagenopathy is preferably collagenopathy type I, II or XI.

In another embodiment, the *D. biflorus* extract is for use as a medicament for treatment or prevention of a disease caused by impaired or reduced cell metabolism.

In another embodiment, the *D. biflorus* extract is for use as a medicament for treatment or prevention of a disease caused by oxidative stress.

In another embodiment, the *D. biflorus* extract is for use as a medicament for treatment or prevention of an inflammatory disease. The *D. biflorus extract* can be used for treating or preventing inflammation, especially inflammation induced by UV rays. Such inflammation can be a local inflammatory response and/or a systemic inflammation. For example, the *D. biflorus* extract be used to treat or prevent inflammatory conditions including gingivitis and periodontitis; inflammatory diseases of the skin, including sclerodermatitis, psoriasis and eczema.

In another embodiment, the *D. biflorus* extract is for use as a medicament for restoring the integrity of collagen containing tissue, especially of the skin, e.g. after mechanical wounding or burning, by enhancing the collagen production. Wound healing is especially preferred. Collagens are widely employed in the construction of artificial skin substitutes used in the management of severe burns. Enhancing the collagen production of the patient's own cells will support the healing process of severed skin, e.g. after mechanical wounding (for example, after surgery), burning, or damage caused by environmental factors like chemical burns.

In another embodiment the *D. biflorus* extract is for use as a medicament for restoring the integrity of tissue, especially of the skin, by enhancing the cell metabolism and thus the metabolic rate of the cell. This may lead to a forced rate of cell division and tissue rejuvenation, e.g. after mechanical wounding or burning. Wound healing is especially preferred. Enhancing the metabolic rate of the patient's own cells will support the healing process of severed skin, e.g. after mechanical wounding (for example, after surgery), burning, or damage caused by environmental factors like chemical burns.

When administered to an animal, the *D. biflorus* extract is typically administered as a component of a composition that comprises a pharmaceutically acceptable carrier or excipient. The *D. biflorus* extract or pharmaceutical composition according to present invention is typically administered topically by bringing it into contact with the skin of the patient. This can be achieved, for example, by applying a wound dressing, a semisolid or liquid formulation (e.g. a spray, a cream, an ointment), a patch or an adhesive bandage. However, the *D. biflorus* extract may also be administered by any other convenient route, for example, by absorption through epithelial or mucocutaneous linings. The mode of administration is left to the discretion of the practitioner.

The amount of the *D. biflorus* extract or composition comprising said extract to be administered, as well as the dosing schedule necessary to provide the desired preventive or therapeutic effect will be dependent on the bioavailability of the active ingredients, the disorder being treated, and other factors that will be apparent to those skilled in the art. For topical application, it is convenient to provide the active compound in a pharmaceutical preparation at a concentration which will facilitate easy application of the appropriate dosage, i.e., the concentration should be chosen so that the volume of preparation to be applied is not too great or too small.

The amount of the *D. biflorus* extract that is effective as a medicament can be determined by standard clinical techniques. In addition, *in vitro* or *in vivo* assays can optionally be employed to help identify optimal dosage ranges. The precise dose to be employed will also depend on the route of administration and the seriousness of the condition, and can be decided according to the judgment of a practitioner and/or the subject's physical condition.

In cases wherein the *D. biflorus* extract or composition according to present invention is administered in order to prevent tissue damage, e.g. by oxidative stress induced by external factors, the *D. biflorus* extract or composition will advantageously be administered to the tissue prior to the contact with said factors.

The properties and preferred features of the *Dolichos biflorus* extract according to present invention and the pharmaceutical composition comprising a *Dolichos biflorus* extract according to present invention described in the previous sections specify the properties and preferred features of the *Dolichos biflorus* extract and composition to be used as a medicament in the therapeutic use and method.

In a specific embodiment, the present invention pertains to the therapeutic use of a *D. biflorus* extract or a composition comprising such extract in enhancing collagen production, especially collagen production in skin and/or skin cells. Preferably, the production of collagen I, III, IV, V, VI, VII, XII, XIV, XVI, XVIII and/or XIX, more preferably of collagen I and/or V, and even more preferably of both collagen I and V is enhanced.

In a particular aspect, the therapeutic use of a *D. biflorus* extract in skin treatment according to present invention is based on the ability of said extract to enhance both collagen I and V production. This may lead to the *de novo* synthesis of a high quality collagen network.

The *D. biflorus* extract may be used in its pure form or as part of a composition comprising further ingredients. It is preferentially used in a composition, particularly at a concentration from about 0.0001 to about 10% and more preferentially from about 0.001 to about 5%, even more preferentially from about 0.01 to about 2% by weight based on the total composition weight.

The invention also relates to a method for causing in a cell, particularly in a skin cell, the effects of the *D. biflorus* extract on a cell described in this section and the previous sections. The method comprises the step of contacting a cell with an effective amount of a *D. biflorus* extract. This method can be used *in vitro,* but is especially useful *in vivo.* The method is performed by contacting a cell with an effective amount of a *D. biflorus* extract. Preferably, the method is used to enhance collagen production by a cell.

Where a cell is contacted with the *D. biflorus* extract *in vitro,* the amount of the *D*. *biflorus* extract will typically range from about 0.00001 to about 10 % (w/v), preferably from about 0.0001 to about 1 % (w/v), more preferably from about 0.001 to 0.5 % (w/v), even more preferably from about 0.003 to 0.1 % (w/v), including ranges from about 0.003 to about 0.01 % (w/v) and from about 0,03 to about 0.1 % (w/v) dry matter of extract in a solution or suspension.

Further features of the extract and the composition comprising said extract according to present invention which are suitable for the therapeutic use described herein are set forth in the previous sections.

The invention also relates to a process for preventing or treating the conditions and diseases described above, which process comprises administering a therapeutically effective amount of the *D. biflorus* extract according to present invention to a patient in need thereof. Said process comprises the step of applying a *D. biflorus* extract according to present invention or a pharmaceutical composition according to present invention to the patient, preferably to the patient's skin. Said application is typically a topical application. The *D. biflorus* extract or pharmaceutical composition is applied in an effective amount and/or period of time which is sufficient to achieve the effects underlying the therapeutic use of the *D. biflorus* extract according to present invention.

The present invention is further described in more detail by reference to the following examples. It should be understood that these examples are for illustrative purpose only and not to be construed as limiting the invention.

### Examples

In the following examples, standard techniques of molecular biology and cell culture were used that are described in various textbook publications, e.g. Sambrook et al. (2001), Molecular Cloning: A Laboratory Manual, 3rd edition, Cold Spring Harbor Laboratory Press, or Ausubel et al. (1987), Current Protocols in Cell Biology, edition as of 2002, Wiley Interscience. Unless otherwise indicated, all enzymes, cells, reagents, devices and kits were used according to the manufacturers' instructions and the chemicals were of the highest available purity grade.

### Example 1: Hydro-glycolic extract of Dolichos biflorus seeds

100 g of dried *Dolichos biflorus* seeds were grinded. 700 ml of 1,3-butyleneglycol and 300 ml of distilled water were added to the flour obtained, and extraction was performed under agitation 1 h at 80°C. The suspension was cooled down at room temperature and centrifuged to obtain a clear supernatant, which was filtrated on paper to obtain 828 ml of a yellowish clear liquid, with 0.75 wt.-% of dry matter in the hydroglycolic extract as determined with a halogen moisture analyzer (Mettler Toledo).

### Example 2: Hydro-methanolic extract of Dolichos biflorus seeds

50 g of dried *Dolichos biflorus* seeds were grinded. 800 ml of methanol and 200 ml of distilled water were added to the flour obtained, and extraction was performed under agitation 20 h at room temperature. After 1 h of decantation, the suspension was filtrated on paper to obtain a yellowish clear liquid. After removal of the methanol under vacuum, the liquid extract was freeze-dried to yield 3.8 g of a brownish powder.

### Example 3: Hydro-ethanolic extract of Dolichos biflorus seeds for 20 h

50 g of dried *Dolichos biflorus* seeds were grinded. 700 ml of ethanol and 300 ml of distilled water were added to the flour obtained, and extraction was performed under agitation 20 h at room temperature. After 1 h of decantation, the suspension was filtrated on paper to obtain a yellowish clear liquid. After removal of the ethanol under vacuum, the liquid extract was freeze-dried to yield 4.8 g of a brownish powder.

### Example 4: Hydro-ethanolic extract of Dolichos biflorus seeds for 1 h

100 g of dried *Dolichos biflorus* seeds were grinded. 700 ml of ethanol and 300 ml of distilled water were added to the flour obtained, and extraction was performed under agitation 1 h at room temperature. After 1 h of decantation, the suspension was filtrated on paper to obtain a yellowish clear liquid. After removal of the ethanol under vacuum, the liquid extract was freeze-dried to yield 7.9 g of a brownish powder.

### Example 5: Hydro-ethanolic extract of Dolichos biflorus seeds under reflux

100 g of dried *Dolichos biflorus* seeds were grinded. 630 ml of ethanol and 270 ml of distilled water were added to the flour obtained, the suspension was heated at 70°C, and extraction was performed under agitation 1 h when reflux was obtained. The suspension was cooled down at room temperature, and was filtrated on paper to obtain a yellowish clear liquid. After removal of the ethanol under vacuum, the liquid extract was freeze-dried to yield 9 g of a brownish powder.

### Example 6: Aqueous extract of Dolichos biflorus seeds

150 g of dried *Dolichos biflorus* seeds were grinded. 850 ml of distilled water were added to the flour obtained, the pH was adjusted to 4.5 with sulfuric acid 4N, and extraction was performed under agitation 1 h at room temperature. The suspension was centrifuged and filtrated on paper to obtain a clear liquid with 2.6 wt-% of dry matter in the aqueous extract as determined with a halogen moisture analyzer (Mettler Toledo). After addition of 2.6 wt.- % of maltodextrine, the solution was spraydried to yield 15.4 g of a beige powder.

### Example 7: Enzymatic hydrolysate of aqueous extract of Dolichos biflorus seeds

150 g of dried *Dolichos biflorus* seeds were grinded. 850 ml of distilled water were added to the flour obtained, the pH was adjusted to 7.5 with sodium hydroxide 4N, and extraction was performed under agitation at room temperature. After 1 h, 2 g of alcalase^{®} 2.4 L FG (Novozymes) were added, and the extract was hydrolyzed during 2 h 30 min at 50°C. The enzyme was inactivated by heating the suspension 15 minutes at 85°C. After cooling at room temperature, the suspension was centrifuged and filtrated on paper to obtain a clear liquid. The liquid hydrolyzed extract was freeze-dried to obtain 47.2 g of a beige powder.

### Example 8: Regenerative and revitalizing effects on human fibroblasts in culture

The purpose of these tests was to evaluate the revitalizing and regenerating activities of the extracts prepared in the preceding examples on human fibroblasts cultured *in vitro.*

The efficacy test on growing human fibroblasts ("growth test") was carried out in order to evaluate the regenerating and the growth factor like activities. In said test, human fibroblasts (cell line MRC5) were inoculated in standard medium for cell culture (DMEM = Dulbecco Minimum Essential Medium, Invitrogen N°41965-039, with 10 % (v/v) foetal calf serum (FCS)). After an incubation of 1 day at 37°C and 5% CO₂, growth medium was exchanged for standard medium (DMEM without FCS) with different concentrations of the *D. biflorus* extracts. After an incubation of 3 days, the number of viable cells was determined on cell homogenates (in a solution of NaOH 0,1 N) by measurement of the level of protein according to the Bradford's method (Bradford et al. (1976) Anal. Biochem. 72:248-254).

The survival efficacy test ("survival test") was carried out on human fibroblasts, to evaluate the regenerating and the revitalizing activities by measuring the cellular viability of human fibroblasts cultured in the presence of *Dolichos biflorus* extracts. Human fibroblasts (cell line MRC5) were inoculated in standard medium of cell culture with FCS as described above. After an incubation of 3 days the cells became quiescent and the growth medium was exchanged for standard medium (DMEM without FCS) with a range of concentrations for each substance to be tested. After a subsequent incubation of 3 days at 37°C, the effects on cultured cells metabolism were determined by measuring on cell homogenates (in a solution of NaOH 0,1 N) the cellular levels of proteins according to the Bradford's method, ATP content (enzymatic method according to Vasseur (1981) Journal francais Hydrologie 9:149-156) and cellular DNA (determined by staining of DNA with Hoechst 33258 reagent and recording the fluorescence at 455 nm with an excitation at 365 nm according to Rao, J., and Otto, W.R. Fluorimetric DNA assay for cell growth estimation, Anal. Biochem. 207:186-192; 1992).

The assays have been carried out in triplicate and repeated three or four times, the results were calculated referring to a standard range for proteins, ATP and DNA and presented in % referring to the control (standard medium without addition).

**Table 1: Results (%/control) of growth test and survival test. Dose: amount of D. biflorus extract powder added to the culture medium**

| Extract | Growth test | | Survival test | | | |
|---|---|---|---|---|---|---|
| | Dose % w/v | Protein level | Dose % w/v | DNA level | ATP level | Protein level |
| Control (untreated) | / | 100 | / | 100 | 100 | 100 |
| Extract according example 2 | 0.1 | 254 | 0.1 | 166 | 212 | 181 |
| Extract according example 3 | 0.1 | 234 | 0.1 | 140 | 205 | 181 |
| Extract according Example 4 | 0.1 | 218 | 0.03 | 186 | 203 | 190 |
| Extract according Example 5 | 0.1 | 230 | 0.1 | 170 | 247 | 180 |

These results show that *Dolichos biflorus* seed extracts improved cell growth and cell metabolism, and thus have a good potential to stimulate skin renewal, e.g. in wound healing.

### Example 9: Stimulation of collagen I production

The purpose of this assay was the evaluation of the potential of *Dolichos biflorus* seed extract to increase the level of collagen I. Human dermal fibroblasts (from skin of aesthetic surgery) were cultivated in growth medium (DMEM Invitrogen N°41965-039 with FCS at 10 % (v/v)) for 24 hours at 37°C. Then the medium was exchanged for standard medium (DMEM Invitrogen N°41965-039 with FCS at 1 % (v/v)) containing the products to be tested and incubated for 72 hours at 37°C. The amount of cellular proteins was determined on cell homogenate (in a solution of NaOH 0.1 N) by the Bradford's method. The quantity of synthesized type I collagen was determined by measuring the amount of released collagen peptides in cell culture medium by an ELISA method (kit Metra N° from Osteomedical).

The results are shown in table 2. They are expressed in % protein referring to the control (= standard medium with cells).

**Table 2: Effect of D. biflorus extracts on cell proteins and on release of collagen I. Results in % protein against control (mean value of 3 assays in triplicate). Dose: % w/v of extract/cell culture medium.**

| Test substance | Dose [% w/v] | Cell proteins | Released type I collagen |
|---|---|---|---|
| Control | - | 100.0 | 100.0 |
| Vitamin C | 20 µg/ml | 77.3 | 118.3 |
| Extract according to example 3 | 0.1 | 95.1 | 117.6 |

These results show that *Dolichos biflorus* seed extract distinctly enhanced the level of released collagen I by dermal human fibroblasts as well as Vitamin C.

### Example 10: Stimulation of expression of collagen V encoding genes in cultured human fibroblasts

This test was performed to evaluate the stimulation efficacy of a *Dolichos biflorus* extract according to example 3 on the expression of collagen V encoding genes in human fibroblasts. As 2 genes are responsible of the production of collagen V, namely the genes COL5A1 and COL5A2, both gene expressions were analysed.

Human dermal fibroblasts (obtained from plastic surgery) were inoculated in standard DMEM medium of cell culture with 10% FCS (Invitrogen). When cell confluence was arisen, the substances to be tested were introduced in new DMEM without FCS. Fibroblasts were incubated during 24 h with the test substances, then total RNA was extracted using a commercial extraction kit (Nucleospin™ RNA II, Macherey-Nagel™, France). Quantification and quality control of total RNA were performed by measuring fluorescence at 260 and 280 nm.

Specific qRT-PCR methodologies were performed to analyze either *COL5A1* or *COL5A2* gene expression. In both cases, target mRNA level was compared to the rate of a housekeeping gene mRNA, whose expression is not strongly modified by treatments: the translation elongation factor 1-alpha (EF1α).

The reverse transcription step was performed with a reverse transcription (RT) kit (ThermoScript™ RT-PCR System, Invitrogen, France) using 0.1 µg of total RNA in a 20 µl final volume. The quantitative PCRs were performed on 2 µl of the RT mix with specific primers in a 20 µl final volume, according to the manufacturer's informations (LightCycler^{®}480 SYBR Green I Master, Roche, France). Each analysis was repeated twice. Results, expressed as percentage RNA compared to the control, are the mean of three independent assays (table 3).

TGF-β1 was used as the positive reference. This growth factor is a strong transcription activator of genes encoding the dermal major collagens I and III. It was validated that TGF-β1 also increases levels of COL5A1 and COL5A2 mRNA. As this growth factor is highly labile, a carrier protein was added with TGF-β1 to increase its stability. A control treatment with only the carrier protein was performed. This protein had only a very weak effect on the levels of collagen V mRNA.

**Table 3: RNA levels of COL5A1 and COL5A2 after incubation with test substances [%/control].**

| Test substance | COL5A1 | COL5A2 |
|---|---|---|
| control | 100 | 100 |
| Extract according to example 3 at 0.03 % w/v | 185 | 135 |
| Reference (TGF-β1) at 10 ng/ml | 567 | 171 |
| Reference control | 99 | 95 |

These results clearly show that treatment with *Dolichos biflorus* seed extract has increased the levels of both mRNAs coding for type V collagen in human dermal fibroblasts. This effect induces an increase of the synthesis of collagen V. Collagen V is implicated in the correct formation and structure of collagen I fibers. Thus, in addition to its effect on the rate of production of collagen I, *Dolichos biflorus* seed extract positively influences the structure of these neo-synthesized collagen I fibers.

### Example 11: UVA cytophotoprotection, anti-oxidative effect

The cytoprotection against UVA irradiation was evaluated by a test on human fibroblasts because UVA rays penetrate through the epidermis into the dermis where they induce oxidative stress, mainly by activation of photosensitising biological components, which catalyse the formation of reactive oxygen species (ROS) like anion superoxide, hydrogen peroxide and singlet oxygen, and lipoperoxydation of the cell membrane (Dalle Carbonare M, Pathak MA (1992) J Photochem & Photobiol 14:1-2, 105-124). The lipoperoxides formed after UVA irradiation are decayed into malondialdehyde which can form cross-links between many biological molecules like proteins resulting in inhibition of enzymes and damage to nucleic acids with risk of mutagenesis. Thus, oxidative stress was evaluated *in vitro* by measuring of the level of released malondialdehyde (MDA).

Human fibroblasts (cell line MRC5) were inoculated with growth medium as described above (DMEM (Invitrogen N°41965-039) + FCS) and incubated 3 days at 37°C, with 5% CO₂. The growth medium was then exchanged with medium containing the test substance and incubated 2 days at 37°C with 5% CO₂. After exchange of medium with balanced salt solution (Hank's BSS SIGMA H1387), the cell culture was irradiated by UVA 20 J/cm². Cell proteins and GSH (glutathione) were measured respectively using Bradford's reagent (according to MM. Bradford: A rapid and sensitive method for the quantitation of microgram quantities of protein utilizing the principle of protein-dye binding, Anal, Biochem 72:248-254 (1976)) and by the OPT (ortho-phthalaldehyde) method (according to Hissin PJ, Hilf R.: A fluorometric method for determination of oxidized and reduced glutathione in tissues, ANALYTICAL BIOCHEMISTRY 74:214-26 (1976)), and MDA released in the supernatant was spectrophotometrically determined using TBA (thiobarbituric acid) reagent (according to Morliere P et al., UV-A induced lipid peroxydation in cultured human fibroblasts, Biochim. Biophys. Acta, 1084, 3:261-269 (1991)).

**Table 4: MDA release after UVA irradiation. Results in % against control (mean value of 2 assays in triplicate). Dose: % w/v of extract/cell culture medium.**

| Test substance | Dose %w/v | MDA released | Cell proteins |
|---|---|---|---|
| Control (not irradiated) | - | 0 | 100 |
| Control UVA irradiated ( 20 J/cm²) | - | 100 | 108 |
| Extract according example 2 UVA irradiated (20 J/cm²) | 0.003 | 63 | 142 |
| | 0.01 | 58 | 171 |

Higher doses of 0.03 and 0.1 % (w/v) extract according to Example 2 were also tested once in triplicate and were also highly effective.

The UVA irradiation has induced a release of MDA in the control. After incubation of the fibroblasts with *Dolichos biflorus* extract, the MDA release was reduced in comparison to the untreated control. Thus, a protection of cells against UVA-induced oxidative damages was obtainable by *D. biflorus* treatment.

### Example 12: UVB-cytophotoprotection.

The arachidonic acid cascade is an important mechanism of the cutaneous inflammation. This cascade may be induced by several factors, particularly by UVB irradiation. UVB induces the inflammatory response by activation of phospholipase A2 (PLA2), which releases arachidonic acid from the cell membrane (De Leo VA et al., Photochemistry and Photobiology (1985) 41:51-56). Subsequently, cyclooxygenases transform arachidonic acid into prostaglandins (PG), which are secreted out of cells. The binding of certain prostaglandins (PGE₂) to specific skin receptors is followed by redness and swelling of the human skin. In cultured human cells, the UVB effects on the cell membrane are associated with a release of a cytoplasmic enzyme, the Lactate Dehydrogenase (LDH), into the supernatant medium (Bonnekoh B et al. (1990) Dermatological Research 282:325-331).

Human keratinocytes (Cell line A431) were inoculated with growth medium (DMEM+FCS) (1 ml per well in plates with 12 wells), and incubated 3 days at 37°C, with 5% CO₂. The growth medium was then exchanged with balanced salt solution containing the substance to be tested, and the cell culture was irradiated by UVB 50 mJ/cm² (DUKE GL40E lamp). After 1 day of incubation at 37°C with 5% CO₂, LDH (kit Roche N° 1644793) and PGE₂ (kit Oxford Biomedical N°EA02 via Euromedex) released into the medium were determined, and cellular DNA was measured using the fluorescent probe Hoechst 33258 reagent (according to Rao, J., and Otto, W.R. Fluorimetric DNA assay for cell growth estimation. Anal. Biochem. 207:186-192 (1992)) to determine the cell viability.

**Table 5: LDH and PGE₂ release after UVB irradiation. Results in % against control (mean value of 2 or 3 assays in triplicate). Dose: % w/v of extract/cell culture medium.**

| Product | Dose % w/v | Keratinocyte DNA | LDH released | PGE₂ released |
|---|---|---|---|---|
| Control (not irradiated) | - | 100 | 0 | 0 |
| Control / UVB (50 mJ/cm²) | - | 23 | 100 | 100 |
| Extract according example 2 / UVB (50 mJ/cm²) | 0.03 | 35 | 47 | 43 |
| | 0.1 | 45 | 40 | 29 |

The UVB irradiation of the control induced an inflammation with a release of PGE₂ and with cell membrane injury as demonstrated by the release of LDH activity in the medium, and a decrease of keratinocyte cell number (decrease of around 77 % of cell DNA). After incubation of the keratinocytes with *Dolichos biflorus* extract and UVB irradiation, an increase of viable cells and a decrease of released LDH and PGE₂ were obtained in comparison to the UVB irradiated control. These results demonstrate the ability of *Dolichos biflorus* extract to protect skin cells from the damages induced by UVB irradiation and inflammation.

## Claims

1. A *Dolichos biflorus* extract for use as a medicament for enhancing collagen production in the skin.

2. The *Dolichos biflorus* extract according to claim 1, wherein the collagen is a collagen selected from the group consisting of collagen I, III, IV, V, VI, VII, XII, XIV, XVI, XVIII, XIX and any combination thereof.

3. The *Dolichos biflorus* extract according to claim 1 or 2, wherein the collagen is collagen I and/or V.

4. The *Dolichos biflorus* extract according to any one of claims 1 to 3, wherein the medicament is for treating a collagen disorder or for wound healing.

5. The *Dolichos biflorus* extract according to any one of claims 1 to 4, wherein the *Dolichos biflorus* extract additionally acts as a UV-protective factor, an antioxidant, and/or a metabolism enhancer.

6. The *Dolichos biflorus* extract according to any one of claims 1 to 5, which
(i) is an extract of a seed or a seed containing plant part, and/or
(ii) is an alcoholic or hydroalcoholic extract.

7. Use of the *Dolichos biflorus* extract as defined in any one of claims 1 to 6 for the preparation of a medicament for enhancing collagen production in the skin.

8. A pharmaceutical composition comprising the *Dolichus biflorus* extract as defined in any one of claims 1 to 6.

9. A method for preparing the *Dolichus biflorus* extract as defined in any one of claims 1 to 6, comprising a step wherein a *Dolichus biflorus* whole plant or plant part is extracted, preferably by alcoholic or hydroalcoholic extraction.
